# EUROPÄISCHE PATENTSCHRIFT

(11) **EP 0 809 709 B1**
(45) Veröffentlichungstag und Bekanntmachung des Hinweises auf die Patenterteilung: **10.04.2002**
(21) Anmeldenummer: 96904082.3
(22) Anmeldetag: 14.02.1996
(51) Int. Cl.: C12Q 1/26, C12Q 1/66, G01N 21/76, G01N 33/53, G01N 33/58, G01N 33/72

(54) **STARTER-KIT**
STARTER KIT
NECESSAIRE D'AMOR AGE

(30) Priorität: 17.02.1995 DE 19505393
(43) Veröffentlichungstag der Anmeldung: 03.12.1997
(73) Patentinhaber: Byk-Sangtec Diagnostica GmbH & Co. KG, 63128 Dietzenbach (DE)
(72) Erfinder: MARKOWITZ, Gerd, D-63110 Rodgau (DE); LENTFER, Dierck, D-63110 Rodgau (DE)
(74) Vertreter: Rupp, Herbert, Dr.
(86) Internationale Anmeldenummer: EP9600628
(87) Internationale Veröffentlichungsnummer: WO9625516

(56) Entgegenhaltungen:
- EP-A- 0 186 751
- CHEMICAL ABSTRACTS, Band 107, Nr. 5, ver!ffentlicht 1987, 03 August, (Columbus, Ohio,USA), P. JONES et al. "Kinetics and mechanism of catalysis by ferrihemes in the chemi- luminogenic oxidation of luminol by hydrogen peroxid", Seite 669, Spalte 1, Nr. 39 496u; & Photochem. Photobiol. 1987, 45(2), 283-9.
- CHEMICAL ABSTRACTS, Band 103, Nr. 25, ver!ffentlicht 1985, 23 Dezember, (Columbus, Ohio, USA), J.E. FREW et al."Assay of some clinically important reductants by a chemi- luminescence-delay technique", Seite 479, Spalte 2, Nr. 210 411f; & Anal. Lett. 1985, 18(B13), 1579-92.

## Beschreibung

### Technisches Gebiet

Die Erfindung betrifft einen Starter-Kit zur Initiierung der Lichterzeugungsreaktion durch Oxydation von lumineszierenden Molekülen in analytischen Tests.

### Stand der Technik

Licht emittierende chemische Reaktionen finden bei analytischen Tests eine vielfältige Anwendung. In der klinischen Analytik haben sie insbesondere in Immunoassays, beim Protein-blotting und bei DNA-Proben eine weite Anwendung gefunden. Zu den erfolgreichsten Tests gehören solche, bei denen als lumineszierende Moleküle Isoluminolderivate und Acridiniumderivate eingesetzt werden, die in alkalischer Lösung unter Verbrauch von Peroxid Energie in Form von Lichtquanten abgeben. Diese Reaktion ist besonders bei Isoluminolderivaten einer sehr langsamen Kinetik unterworfen. Damit in vernünftiger Zeit eine meßbare Lichtmenge freigesetzt wird, werden Katalysatoren zugesetzt. Diese Katalysatoren enthalten meist komplexiertes Eisen. Neben Blutlaugensalzen werden Verbindungen wie Microperoxidase, Hemin, Hematin oder Enzyme wie Catalase und Meerrettichperoxidase in der Literatur als geeignet beschrieben. Insbesondere Catalase und Microperoxidase wurden in kommerziellen Systemen schon mit Erfolg verwendet. Aus P. Jones et al. (1987), Photochem. Photobiol. 45, 283-289 ist die Verwendung von Deuteroferrihäm als Katalysator bei der Lichterzeugungsreaktion durch Oxydation von Luminol mit Wasserstoffperoxid bekannt. Bei den Isoluminolderivaten haben sich ABEI (Aminobutylethylisoluminol) oder ABENH (Aminobutylethylnaphthalindicarbonsäurehydrazid) durchgesetzt, da sie sich im Gegensatz zu Luminol ohne nennenswerten Verlust an Quantenausbeute problemlos an Haptene oder Proteine koppeln lassen.

Zur Messung der Lumineszenz werden in einem Meßgerät geeignete Reagenzlösungen zu einer das Label in unbekannter Menge enthaltenden Lösung pipettiert und die Lumineszenz sofort gemessen. Die Kinetik der Lichtreaktionen beider Substanzklassen verläuft unter optimierten Bedingungen in wenigen Sekunden.

Mit Bekanntwerden der Lumineszenz in der Literatur wurde ursprünglich zur Initiierung der Lumineszenz das Verfahren angewendet, drei Lösungen zu pipettieren: Natronlauge, Katalysator und abschließend verdünnte Wasserstoffperoxidlösung als Oxidationsmittel [J. Clin. Chem. Clin. Biochem., 21, 789 (1983)]. Mit der zunehmenden Kommerzialisierung der Lumineszenz in Immunoassays wurde eine Vereinfachung der Lichterzeugung erreicht durch Verwendung einer alkalischen Peroxidlösung (DE 3439742). Die beschriebene alkalische Peroxidlösung ist für die Verwendung in Immunoassays innerhalb eines gegebenen Zeitraumes nach dem Ansetzen ausreichend stabil, so daß nach Zugabe des Katalysators durch Injektion der alkalischen Peroxidlösung in die Meßkammer die Lumineszenzreaktion ausgelöst wird. In einer optimierten Variante, die von der Anmelderin seit einigen Jahren kommerziell verwendet wird, läßt sich die Lichtausbeute dadurch steigern, daß erst die alkalische Peroxidlösung pipettiert wird und dann der Katalysator die Lichtreaktion startet. Die Lichtausbeute wird dadurch nochmal deutlich gesteigert. Bei beiden Varianten ist die Konzentration der verwendeten Natronlauge recht hoch (ca. 1 m), da die Lichtausbeute mit steigender Konzentration zunimmt. Die Kombination der beiden Lösungen aus Katalysator und Peroxid bezeichnet man üblicherweise als Starter-Kit.

Ein Hauptproblem der zur Lichterzeugung verwendeten Starter-Kits besteht in ihrer begrenzten Haltbarkeit. Dies hat zur Folge, daß sie in keinem Fall über mehrere Tage ungekühlt in einem automatischen Meßsystem verbleiben können. Aus diesem Grund sollten die Lösungen eines Starter-Kits möglichst am gleichen Tag vollständig aufgebraucht werden oder aber zwischenzeitlich bei 2-8°C im Kühlschrank aufbewahrt werden.

Stabilitätsuntersuchungen haben gezeigt, daß die flüssigen Gebrauchslösungen selbst unter optimalen Aufbewahrungsbedingungen nur maximal 14 Tage verwendbar sind. Nach dieser Zeit ist besonders bedingt durch die bekannte Licht- als auch Temperaturempfindlichkeit des Wasserstoffperoxids in alkalischer Lösung zum einen eine deutliche Abnahme des Lumineszenzsignals und zum anderen ein Ansteigen des unspezifischen Reagenzienleerwertes zu beobachten. Somit wird das Rausch-Signal-Verhältnis mit zunehmender Alterung der Gebrauchslösungen deutlich schlechter, was ihre Verwendung im Routinebetrieb oder in einem automatischen Immunoassayanalyzer deutlich einschränkt.

Deshalb werden Starterlösungen auch nicht in gebrauchsfertiger Form in kommerziellen Testsystemen angeboten, sondern müssen vor der eigentlichen Lichtmessung vom Anwender in die gebrauchsfertige Form gebracht werden. Dies bedeutet zum einen einige notwendige Pipettierschritte oder das Auflösen einer Perhydridtablette (Harnstoffperoxid) in dem entsprechenden Lösungsmittel, zum anderen eine mögliche Fehlerquelle bei der manuellen Vorbereitung einer Lumineszenzmessung durch das Bedienungspersonal.

Zum technischen Hintergrund wird auf EP-B 0186751 hingewiesen.

### Beschreibung der Erfindung

Die Aufgabe der vorliegenden Erfindung wird darin gesehen, einen Starter-Kit zur Initiierung der Lichterzeugungsreaktion durch Oxydation von lumineszierenden Molekülen in Gegenwart von Katalysatoren in analytischen Tests so zu verbessern, daß die beiden Komponenten in gebrauchsfertigen Lösungen zur Verfügung gestellt werden können, die über mehrere Monate hinweg stabil bleiben. Ein weiteres Ziel der Erfindung ist darin zu sehen, die beiden Komponenten eines Starter-Kits so zu gestalten, daß sie ohne zusätzliche Vorbereitungsschritte über längere Zeit direkt in automatischen Lumineszenz-Analysegeräten einsetzbar sind.

Es wurde nun überraschenderweise gefunden, daß durch ein Starter-Kit, umfassend als Oxidationsmittel eine neutral bis schwach sauer reagierende wäßrige Lösung mit 0,01 bis 0,1 Gew.-% Wasserstoffperoxid und als Katalysator eine alkalische wäßrige Lösung von Deuteroferrihäm mit einem pH-Wert über 12, diese Aufgaben gelöst werden können.

Der neue Starter-Kit kann direkt in automatischen Lumineszenz-Analysegeräten eingesetzt werden. Durch die mit dem neuen Starter-Kit erreichbare beschleunigte Kinetik kann die Zeitspanne für die Lichtreaktion verkürzt werden, was insbesondere in vollautomatischen Analyzersystemen einen erhöhten Probendurchsatz ermöglicht.

Die vorteilhaften Eigenschaften des neuen Starter-Kits sind überraschend, da nicht erwartet werden konnte, daß organische Verbindungen über Monate in ca. 1 m Natronlauge stabil sein würden. Tatsächlich zeigte sich die bis dahin bevorzugt verwendete Microperoxidase als erfindungsgemäß nicht geeignet. Mit Deuteroferrihäm (DFH) konnte jedoch eine Verbindung gefunden werden, die die Stabilitäts- und lichtmeßtechnischen Anforderungen voll erfüllt. Die Synthese von DFH erfolgt durch Umsetzung von Hemin mit Resorcin nach der Vorschrift von P. Jones et al. aus J. of Chemical Education 64, 70 (1987). Das nach dem Umkristallisieren erhaltene Produkt erfüllt die erfindungsgemäßen Anforderungen.

Gegenstand der Erfindung ist daher ein Starter-Kit zur Initiierung der Lichterzeugungsreaktion durch Oxydation von lumineszierenden Molekülen in analytischen Tests umfassend Oxidationsmittel und Katalysatoren, der dadurch gekennzeichnet ist, daß als Oxidationsmittel Wasserstoffperoxid in einer Konzentration von 0,01 bis 0,1 Gew.-% in destilliertem Wasser vorliegt und als Katalysator Deuteroferrihäm in einer Menge von 200 bis 3000 ng/ml in 0,1 bis 2 n wäßriger Natriumhydroxidlösung vorliegt.

Weitere Gegenstände ergeben sich aus den Patentansprüchen.

Das in Verbindung mit der neuen alkalischen Katalysatorlösung zur Lichtmessung verwendete Wasserstoffperoxid kann jetzt in schwach saurer oder neutraler Lösung verwendet werden und ist darin über ein Jahr ohne Aktivitätsverlust in Gebrauchsform haltbar. Als Nebeneffekt kann die Konzentration geringer gehalten werden, was den Reagenzienleerwert zusätzlich deutlich positiv beeinflußt. In der ersten Komponente des erfindungsgemäßen Starter-Kits liegt Wasserstoffperoxid jetzt in einer Konzentration von 0,01 bis 0,1, vorzugsweise 0,03 bis 0,04 Gew.-% in wäßriger Lösung vor. Die erste Komponente wird vorzugsweise hergestellt, indem eine wäßrige Wasserstoffperoxidlösung mit destilliertem Wasser verdünnt wird. In der zweiten Komponente des erfindungsgemäßen Starter-Kits liegt Deuteroferrihäm in einer Konzentration von 200 bis 3000, vorzugsweise 600 bis 1000 ng/ml in 0,01 bis 2 n, vorzugsweise 0,5 bis 1,5 n wäßriger Natriumhydroxidlösung vor. Die Reihenfolge der Injektionen der beiden Starterlösungen hat nur geringen Einfluß auf das Meßergebnis, bevorzugt ist jedoch die Zugabe der Oxidationslösung vor der Katalysatorlösung wegen der höheren Lichtausbeute und der auch gegenüber der herkömmlichen Variante schnelleren Kinetik der Lichtreaktion. Dadurch kann die Meßzeit verringert und der Durchsatz erhöht werden.

Beide gebrauchsfertige Lösungen zeigten über mehrere Monate weder einen Verlust der katalytischen Aktivität noch einen Anstieg des unspezifischen Backgrounds, welcher bei der Verwendung dieser Reagenzien in immunologischen Testsystemen wesentlich für die Empfindlichkeit der betreffenden Immunologischen Testbestecke verantwortlich ist. Durch den Wegfall der Lösungsaufbereitungen sind diese Reagenzien nicht nur einfacher in ihrer Handhabung sondern bergen auch keine mögliche Verwechslungsgefahr mehr während ihrer Präparation.

Die Stabilität der beiden gebrauchsfertigen Komponenten des erfindungsgemäßen Starter-Kits wurde bei Raumtemperatur und bei 37°C am Beispiel eines immunoluminometrischen Assays für PSA (Prostate Specific Antigen) auf dem vollautomatischen Lumineszenzanalyzer LIA-mat S 300 (Byk-Sangtec Diagnostica) untersucht. Selbst nach 6 Monaten ergaben sich weder ein Verlust an Assayempfindlichkeit noch eine Verringerung des maximalen Lumineszenzsignals.

## Patentansprüche

1. Starter-Kit zur Initiierung der Lichterzeugungsreaktion durch Oxydation von lumineszierenden Molekülen in analytischen Tests umfassend Oxidationsmittel und Katalysatoren, **dadurch gekennzeichnet, daß** als Oxidationsmittel Wasserstoffperoxid in einer Konzentration von 0,01 bis 0,1 Gew. % in destilliertem Wasser vorliegt und als Katalysator Deuteroferrihäm in einer Menge von 200 bis 3000 ng/ml in 0,1 bis 2 n wäßriger Natriumhydroxidlösung vorliegt.

2. Starter-Kit nach Anspruch 1, wobei Wasserstoffperoxid in einer Konzentration von 0,03 bis 0,04 Gew. % in destilliertem Wasser vorliegt und als Katalysator Deuteroferrihäm in einer Menge von 600 bis 1000 ng/ml in 0,5 bis 1,5 n wäßriger Natriumhydroxidlösung vorliegt.

3. Verwendung eines Starter-Kits nach Anspruch 1 oder 2 in einem automatischen Lumineszenz-Analysengerät.

4. Verwendung einer neutral bis schwach sauer reagierenden wäßrigen Lösung von Wasserstoffperoxid und einer alkalischen wäßrigen Lösung von Deuteroferrihäm mit einem pH-Wert über 12 zur Initiierung der Lichterzeugungsreaktion durch Oxydation von lumineszierenden Molekülen in analytischen Tests.

5. Verfahren zur Initiierung der Lichterzeugungsreaktion durch Oxydation von lumineszierenden Molekülen in analytischen Tests, **dadurch gekennzeichnet, daß** man zuerst eine neutral bis schwach sauer reagierende wäßrige Lösung von Wasserstoffperoxid und dann eine alkalische wäßrige Lösung von Deuteroferrihäm mit einem pH-Wert von mindestens 12 zugibt.

## Claims

1. Starter kit to initiate luminiferous reaction by oxidation of luminescent molecules in analytical tests comprising oxidants and catalysts, **characterized in that** as oxidant hydrogen peroxide is present in a concentration of from 0.01 to 0.1% by weight in distilled water and as catalyst deuteroferrihaem is present in an amount of from 200 to 3000 ng/ml in 0.1 to 2 N aqueous sodium hydroxide solution.

2. Starter kit according to Claim 1, in which hydrogen peroxide is present in a concentration of from 0.03 to 0.04% by weight in distilled water and as catalyst deuteroferrihaem is present in an amount of from 600 to 1000 ng/ml in 0.5 to 1.5 N aqueous sodium hydroxide solution.

3. Use of a starter kit according to Claim 1 or 2 in an automatic luminescence analysis apparatus.

4. Use of a neutral- to weakly acidic-reacting aqueous solution of hydrogen peroxide and of an alkaline aqueous solution of deuteroferrihaem having a pH above 12 to initiate the luminiferous reaction by oxidation of luminescent molecules in analytical tests.

5. Procedure to initiate the luminiferous reaction by oxidation of luminescent molecules in analytical tests, **characterized in that** first a neutral- to weakly acidic-reacting aqueous solution of hydrogen peroxide is added and then an alkaline aqueous solution of deuteroferrihaem having a pH of at least 12.

## Revendications

1. Kit d'amorçage pour le déclenchement de réactions lumineuses d'oxydation de molécules luminescentes dans des tests analytiques comprenant des agents d'oxydation et des catalyseurs, **caractérisé par le fait que** l'on utilise en tant qu'agent oxydant du peroxyde d'hydrogène en une concentration comprise dans l'intervalle allant de 0,01 à 0,1 % en poids dans l'eau distillée, et en tant que catalyseur du deutéroferrihème en une quantité comprise entre 200 et 3000 ng/ml dans une solution aqueuse d'hydroxyde de sodium à 0,1 - 2 N.

2. Kit d'amorçage selon la revendication 1, dans lequel le peroxyde d'hydrogène est présent en une concentration comprise entre 0,03 et 0,04 % en poids dans de l'eau distillée, et le deutéroferrihème est présent en tant que catalyseur en une quantité comprise entre 600 et 1000 ng/ml dans une solution aqueuse d'hydroxyde de sodium à 0,5 - 1,5 N.

3. Utilisation d'un kit d'amorçage selon la revendication 1 ou 2 dans un appareil automatique d'analyse de luminescence.

4. Utilisation d'une solution aqueuse, neutre à faiblement acide, de peroxyde d'hydrogène et d'une solution aqueuse alcaline de deutéroferrihème ayant un pH supérieur à 12 pour le déclenchement de réactions lumineuses d'oxydation de molécules luminescentes dans des tests analytiques.

5. Procédé de déclenchement de réactions lumineuses d'oxydation de molécules luminescentes dans des tests analytiques, **caractérisé par le fait que** l'on ajoute d'abord une solution aqueuse, neutre à faiblement acide, de peroxyde d'hydrogène, puis une solution aqueuse alcaline de deutéroferrihème ayant un pH au moins égal à 12.
